(19) Europäisches Patentamt / European Patent Office / Office européen des brevets

(11) **EP 4 265 281 B1**

(12) **EUROPEAN PATENT SPECIFICATION**

(45) Date of publication and mention
of the grant of the patent:
**03.12.2025 Bulletin 2025/49**

(21) Application number: **21906344.3**

(22) Date of filing: **01.12.2021**

(51) International Patent Classification (IPC):
*A61L 27/30* (2006.01)      *A61K 6/816* (2020.01)
*A61K 6/84* (2020.01)       *A61L 27/06* (2006.01)
*A61L 27/40* (2006.01)      *A61L 27/54* (2006.01)
*C23C 14/02* (2006.01)      *C23C 14/06* (2006.01)
*C23C 14/48* (2006.01)

(52) Cooperative Patent Classification (CPC):
**A61K 6/84; A61K 6/58; A61K 6/816; A61L 27/06;
A61L 27/30; A61L 27/40; C23C 14/02;
C23C 14/021; C23C 14/0694; C23C 14/48;
C23C 14/5853;** A61L 2400/18; A61L 2430/12

(86) International application number:
**PCT/JP2021/044088**

(87) International publication number:
**WO 2022/130981 (23.06.2022 Gazette 2022/25)**

(54) **COMPOSITE MATERIAL, METHOD FOR MANUFACTURING SAME, AND BIOLOGICAL IMPLANT**

VERBUNDMATERIAL, VERFAHREN ZUR HERSTELLUNG DAVON UND BIOLOGISCHES
IMPLANTAT

MATÉRIAU COMPOSITE, SON PROCÉDÉ DE FABRICATION ET IMPLANT BIOLOGIQUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **15.12.2020 JP 2020207637**

(43) Date of publication of application:
**25.10.2023 Bulletin 2023/43**

(73) Proprietor: **Kyocera Corporation
Kyoto-shi Kyoto 612-8501 (JP)**

(72) Inventors:
• **SAIGA, Kenichi**
  **Kyoto-shi, Kyoto 612-8501 (JP)**
• **HASHIDA, Masahiko**
  **Kyoto-shi, Kyoto 612-8501 (JP)**

• **WATANABE, Kenichi**
  **Kyoto-shi, Kyoto 612-8501 (JP)**
• **KAWASHIMA, Yuki**
  **Kyoto-shi, Kyoto 612-8501 (JP)**

(74) Representative: **TBK
Bavariaring 4-6
80336 München (DE)**

(56) References cited:
**WO-A1-2019/230871      JP-A- 2017 101 275**

• **LIU H Y ET AL: "Effect of fluoride-ion
implantation on the biocompatibility of titanium
for dental applications", APPLIED SURFACE
SCIENCE,, vol. 254, no. 20, 15 August 2008
(2008-08-15), pages 6305 - 6312, XP023177453,
DOI: 10.1016/J.APSUSC.2008.03.075**

**Description**

TECHNICAL FIELD

The present disclosure relates to a composite material, a manufacturing method of the composite material, and a biocompatible implant.

BACKGROUND OF INVENTION

Treatments that restore biological functions by inserting an implant device that includes titanium or the like into the body are widely performed. A challenge in these implant treatments is the reduction of postoperative infections.
For example, as is known in the art, an antibacterial property is exhibited when fluorine ions are implanted in a titanium surface.
LIU H Y ET AL: "Effect of fluoride-ion implantation on the biocompatibility of titanium for dental applications", APPLIED SURFACE SCIENCE, vol. 254, no. 20, 15 August 2008 (2008-08-15), pages 6305-6312, XP023177453, DOI: 10.1016/J.APSUSC.2008.03.075 discloses a study on the effect of fluoride-ion implantation upon the biocompatibility of titanium, wherein fluorine ions are implanted into the smooth surface of pure titanium by using plasma immersion ion implantation technique.

SUMMARY

A composite material according to an aspect of the present disclosure includes a base material and a surface layer located at a surface of the base material. The surface layer contains a compound of titanium and fluorine and a compound of titanium, fluorine, and oxygen. An amount (i.e. abundance) of the compound of titanium and fluorine in the surface layer does not exceed an amount (i.e. abundance) of the compound of titanium, fluorine, and oxygen in the surface layer.
A method for manufacturing a composite material according to an aspect of the present disclosure includes forming a composite material, in which an amount of a compound of titanium and fluorine in a surface layer located at a surface of a base material does not exceed an amount of a compound of titanium, fluorine and oxygen in the surface layer, by any of

(a) implanting $3 \times 10^{17}$ atoms/cm$^2$ or less of fluorine into a base material containing metallic titanium;
(b) implanting fluorine into a base material containing metallic titanium and having an oxide film on a surface;
(c) reacting a fluorine-implanted base material containing metallic titanium with oxygen; or
(d) reacting a base material containing metallic titanium with fluorine and oxygen.

BRIEF DESCRIPTION OF THE DRAWINGS

FIG. 1 is a schematic view of a dental implant according to an embodiment.
FIG. 2 is a graph illustrating amounts of fluorine eluted in Example 1 and in Comparative Example 1.
FIG. 3 is a set of graphs illustrating changes in fluorine concentration before and after immersion in saline in Example 1 and in Comparative Example 1.
FIG. 4 is a set of graphs illustrating changes in fluorine bonding state before and after immersion in saline in Example 1 and in Comparative Example 1.
FIG. 5 is a set of graphs illustrating changes in antibacterial property before and after immersion in saline in untreated titanium as well as in Example 1 and in Comparative Example 1.
FIG. 6 is a graph illustrating relative colony formation rates in untreated titanium and in Example 1.
FIG. 7 is a graph illustrating amounts of fluorine eluted in Example 2 and in Comparative Example 1.
FIG. 8 is a set of graphs illustrating changes in fluorine concentration before and after immersion in saline in Example 2 and in Comparative Example 1.
FIG. 9 is a set of graphs illustrating changes in fluorine bonding state before and after immersion in saline in Example 2 and in Comparative Example 1.
FIG. 10 is a graph illustrating amounts of fluorine eluted in Comparative Example 2 and in Example 3.
FIG. 11 is a set of cross-sectional views of composite materials in Comparative Example 2 and in Example 3 taken by a transmission electron microscope.
FIG. 12 is a set of graphs illustrating changes in fluorine concentration before and after immersion in saline in Comparative Example 2 and in Example 3.

FIG. 13 is a set of graphs illustrating changes in fluorine bonding state before and after immersion in saline in Comparative Example 2 and in Example 3.

FIG. 14 is a graph illustrating antibacterial properties in untreated titanium and in Example 3.

FIG. 15 is a graph illustrating relative colony formation rates in untreated titanium and in Example 3.

DESCRIPTION OF EMBODIMENTS

An embodiment of the present disclosure will be described in detail below. Unless otherwise specified in the present specification, "A to B" representing a numerical value range means "A or more and B or less".

1. Composite Material

According to accepted assumptions, infection can occur approximately six months after orthopedic surgeries or within several years after oral surgeries. In order to solve this problem, the present inventors developed an antibacterial treatment technology by way of fluorine treatment of a titanium material, and achieved a surface treatment with a high antibacterial property and an excellent mechanical characteristic. Meanwhile, it was confirmed that fluorine elutes from the surface of fluorine-treated titanium in an acidic environment. The present inventors found that it is necessary to reduce the elution of fluorine in order to achieve long-term durability that withstands changes in pH in the oral environment caused by in vivo inflammatory reactions as well as eating and drinking.

A composite material according to an embodiment of the present disclosure includes a base material and a surface layer located at a surface of the base material. The surface layer contains a compound of titanium and fluorine and a compound of titanium, fluorine, and oxygen. An amount of the compound of titanium and fluorine in the surface layer does not exceed an amount of the compound of titanium, fluorine, and oxygen in the surface layer. Containing fluorine in the surface layer can impart an antibacterial property. Here, the compound of titanium and fluorine is more easily eluted than the compound of titanium, oxygen, and fluorine. Reducing the amount of the compound of titanium and fluorine can reduce the elution of fluorine. Thus, the antibacterial property can be maintained.

In the present specification, a compound of titanium and fluorine refers to a compound composed of titanium atoms and fluorine atoms. A compound of titanium and oxygen refers to a compound composed of titanium atoms and oxygen atoms. A compound of titanium, fluorine, and oxygen refers to a compound composed of titanium atoms, fluorine atoms, and oxygen atoms. The compound of titanium, fluorine, and oxygen also includes a compound having a structure in which a part of oxygen atoms in a structure composed of titanium atoms and oxygen atoms is substituted with fluorine atoms, as well as a compound having a structure in which a fluorine atom has intruded into a structure composed of titanium atoms and oxygen atoms.

The amount of the compound of titanium and fluorine in the surface layer not exceeding the amount of the compound of titanium, fluorine, and oxygen in the surface layer can be confirmed by measuring the surface layer using X-ray Photoelectron Spectrometry (XPS). A ratio of the peak area of the peak attributed to the compound of titanium and fluorine to the total peak area of the peak attributed to the compound of titanium and fluorine as well as the peak attributed to the compound of titanium, fluorine, and oxygen is calculated from the XPS spectrum. The ratio is 0.5 or less, and may be less than 0.5, or may be 0.4 or less.

The compound of titanium, fluorine, and oxygen may contain at least one selected from the group consisting of TiOF, $TiO_{2-x}F_{2x}$ ($0 < X < 2$), fluorine-substituted $F\text{-}TiO_2$. and fluorine-interstitial $F\text{-}TiO_2$. An example of $TiO_{2-x}F_{2x}$ ($0 < X < 2$) includes $TiOF_2$.

The compound of titanium and fluorine may include TiFX ($1 \leq X \leq 4$). That is, examples of the compound of titanium and fluorine include TiF, $TiF_2$, TiF3 and $TiF_4$.

The composite material may contain a compound of titanium and oxygen. This further reduces elution of fluorine. An example of the compound of titanium and oxygen includes $TiO_2$. A layer containing the compound of titanium and oxygen may be present at the outermost surface of the composite material. A layer containing the compound of titanium and fluorine and/or the compound of titanium, fluorine, and oxygen may be present under the layer containing the compound of titanium and oxygen.

In the composite material, the maximum value of fluorine concentration may exceed 10 atm%, may be 20 atm% or greater, or may be 30 atm% or greater. This can improve antibacterial property. The maximum value of fluorine concentration may be 80 atm% or less, or 70 atm% or less. In the present specification, "fluorine concentration" means a ratio of a number of fluorine atoms per unit volume to the sum of an ideal number of titanium atoms and the number of fluorine atoms per unit volume. Examples of a method for measuring fluorine concentration include secondary ion mass spectrometry (SIMS) and XPS.

The surface layer may have a fluorine concentration of 1 ppm or greater. The surface layer may have a thickness of from 20 to 1100 nm, from 30 to 1000 nm, or from 40 to 900 nm.

The base material may contain pure titanium or a titanium alloy. Examples of pure titanium include Commercially Pure Titanium such as C.P. Grade 2 Titanium. Examples of the titanium alloy include Ti-6Al-4V, Ti-15Mo-5Zr-3Al, Ti-Nb, Ti-6Al-Nb, Ti-6Al-2Nb-1Ta, Ti-30Zr-Mo, Ni-Ti, Ti-3Al-2.5V, Ti-10V-2Fe-3Al, and Ti-15V-3Cr-3Al-3Sn.

The composite material has an amount of fluorine eluted of 1 $\mu$g/cm$^2$ day or less, and may have an amount of fluorine eluted of 0.5 $\mu$g/cm$^2$ day or less, as measured by immersion in an acidic solution. This can maintain an antibacterial property for a longer time. The acidic solution may be, for example, a solution having a pH of 0.6 or less, or a solution having a pH of from 5.5 to 6.0. The acidic solution may be a pH-adjusted saline. For example, the acidic solution may be a saline with citric acid added. The amount of fluorine eluted can be measured by, for example, a method described in Examples.

The composite material may have an antibacterial property. For example, the composite material may have a colony-forming unit count, evaluated by a film adhesion test using Staphylococcus aureus in accordance with JIS Z 2801, that is statistically significantly lower than the colony-forming unit count in untreated titanium. In the present specification, "untreated titanium" means a titanium material that has not been subjected to fluorine implantation and oxidation treatment. Various statistical methods can be used to confirm whether there is a statistically significant difference.

The composite material may be non-cytotoxic. For example, the composite material need not have a relative colony formation rate, evaluated by a direct contact method in accordance with ISO 10993-5, that is not statistically significantly different from the relative colony formation rate of untreated titanium.

2. Manufacturing Method of Composite Material

A method for manufacturing a composite material according to an embodiment of the present disclosure includes forming a composite material, in which an amount of a compound of titanium and fluorine in a surface layer located at a surface of a base material does not exceed an amount of a compound of titanium, fluorine and oxygen in the surface layer, by any of

(a) implanting $3 \times 10^{17}$ atoms/cm$^2$ or less of fluorine into a base material containing metallic titanium;
(b) implanting fluorine into a base material containing metallic titanium and having an oxide film on a surface;
(c) reacting a fluorine-implanted base material containing metallic titanium with oxygen; or
(d) reacting a base material containing metallic titanium with fluorine and oxygen.

By suppressing the amount of fluorine to be implanted or by reacting a base material with oxygen in addition to fluorine as described above, the amount of the compound of titanium and fluorine generated can be lower than the amount of the compound of titanium, fluorine, and oxygen generated. In this way, the above-described composite material can be manufactured.

More than $5 \times 10^{16}$ atoms/cm$^2$ of fluorine may be implanted in the aforementioned (a). This can improve antibacterial property. The fluorine implantation dose in the aforementioned (b) may be from $1 \times 10^{16}$ to $5 \times 10^{17}$ atoms/cm$^2$ or less, or may be from $5 \times 10^{16}$ to $5 \ 10^{17}$ atoms/cm$^2$ The implantation energy may be greater than 30 keV and less than or equal to 80 keV.

The method of implanting fluorine into the base material may be an ion beam implantation method or a plasma-based ion implantation method. The plasma-based ion implantation method is a method of exposing a base material to fluorine-based gas plasma. Examples of the fluorine-based gas include $ArF_2$, $CF_4$, $NF_3$ and $C_2F_6$. The implantation energy in the plasma-based ion implantation method may be from 1 to 50 keV.

Examples of a method of reacting the base material with oxygen include natural oxidation, atmospheric heat treatment, oxygen plasma treatment, oxygen ion implantation, immersion in an acid solution, and anodic oxidation. The temperature in the atmospheric heat treatment may be from 100 to 800°C or from 200 to 300°C.

Anodic oxidation of titanium is a technique of forming an oxide film on a surface of a titanium-based material by applying a potential to the titanium-based material serving as an anode in an aqueous solution or the like. In particular, it is known that by precisely controlling the thickness of the oxide film ranging from several tens of nanometers to several hundreds of nanometers, the oxide film exhibits various colors due to light interference.

The reaction with fluorine and the reaction with oxygen may be carried out sequentially or simultaneously. The aforementioned (b) may include a step of reacting a base material containing metallic titanium with oxygen. In this way, a base material containing metallic titanium and having an oxide film on a surface of the base material may be produced. For example, the oxide film may be formed at a surface of the base material by a method of atmospheric heat treatment, oxygen plasma treatment, oxygen ion implantation, immersion in an acid solution, or anodic oxidation. The aforementioned (c) may include a step of implanting fluorine into a base material containing metallic titanium. In this way, a fluorine-implanted base material containing metallic titanium may be produced.

The manufacturing method may include a step of cleaning the base material. An organic solvent or the like may be used for cleaning. Examples of the organic solvent include ethanol and acetone. Two or more types of organic solvents may be used in combination. The cleaning may be ultrasonic cleaning. The cleaned base material may be vacuum-dried in a desiccator. The step of cleaning the base material may be performed before or after the reaction of the base material and fluorine and/or oxygen.

3. Biocompatible Implant

A biocompatible implant according to an embodiment of the present disclosure includes the composite material described above. Because of that, elution of fluorine is reduced in the biocompatible implant, and thus antibacterial property can be maintained. Examples of the biocompatible implant include dental implants and orthopedic implants. Examples of the orthopedic implants include artificial joints and spinal surgical implants. Examples of the artificial joints include an artificial hip joint, an artificial knee joint, an artificial ankle joint, an artificial shoulder joint, an artificial elbow joint, an artificial finger joint, and an artificial intervertebral disc. Examples of the hip joint include a femoral stem and an acetabular cup. Examples of the spinal surgical implants include spinal fixation instrumentation.

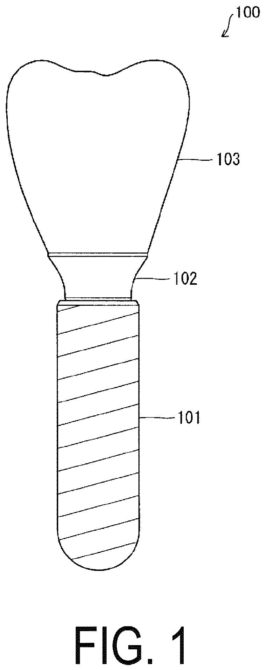

Hereinafter, a dental implant will be described as an example. FIG. 1 is a schematic view of a dental implant 100 according to an embodiment of the present disclosure. The dental implant 100 includes a fixture 101, an abutment 102 mounted to an end portion of the fixture 101, and an artificial tooth 103 mounted to the fixture 101 via the abutment 102.

In the dental implant 100, the fixture 101, the abutment 102, and the artificial tooth 103 may each include the composite material. As described above, the composite material can maintain an antibacterial property. As such, the dental implant 100 can reduce bacterial growth while exhibiting excellent durability against tooth brushing, repeated use, cleaning, and the like.

The fixture 101, the abutment 102, and the artificial tooth 103 may each be composed only of the composite material. In the fixture 101, the abutment 102, and the artificial tooth 103, a portion thereof may be composed of the composite material while the remaining portion may be composed of a material other than the composite material. At least one selected from the group consisting of the fixture 101, the abutment 102, and the artificial tooth 103 may contain the composite material while the remaining members may contain a material other than the composite material. For example, when the fixture 101 and the abutment 102 that are used in an oxygen-deficient environment contain the composite material, the growth of anaerobic bacteria can be expected to be reduced. For example, when the artificial tooth 103 that is exposed in the oral cavity and exposed to the air contains the composite material, the growth of facultative anaerobic bacteria and aerobic bacteria can be expected to reduce. Therefore, the composite material can be applied to the fixture 101, the abutment 102, and the artificial tooth 103 according to the bacterial species whose growth is the target of reduction and the required antibacterial performance.

The surface layer of the composite material may be located at a portion of the dental implant 100 that is likely to be in contact with bacteria. For example, the surface layer of the composite material may be located at surfaces of the fixture 101, the abutment 102, and the artificial tooth 103. Alternatively, the surface layer of the composite material may be located at portions where the fixture 101, the abutment 102, and the artificial tooth 103 are bonded to each other.

4. Other Applications

The composite material described above can also be applied to members other than the biocompatible implant. Because of its antibacterial function that can withstanding fluctuations in the oral environment, the composite material can also be applied to orthodontic wires. In addition, the composite material can maintain the antibacterial function even after repeated cleaning. Therefore, the composite material can be applied to, for example, a surgical instrument, an injection needle, an eyeglass frame, tableware (including portable tableware), a water bottle (for example, the mouthpiece), a kitchen knife, a food factory line, a toilet, a warm water bidet, a faucet, and water and sewage pipes.

EXAMPLES

An example of the disclosure will be described below.

Evaluation Method

Amount of Fluorine Eluted

A composite material of an Example or a Comparative Example was immersed in 10 mL of saline at 37°C for 24 hours. Then, the fluorine ion concentration in the saline was measured to determine the amount of fluorine eluted. A fluorine ion electrode was used to measure the fluorine ion concentration. In order to stably measure the fluorine ion concentration, an ionic strength adjusting agent (citrate buffer) was added to the saline. In the other evaluation methods described below, a citric acid buffer was also added to saline. The amount of fluorine eluted was measured under the following conditions.

Measurement Conditions for Amount of Fluorine Eluted

> Solution for immersion: 0.9% NaCl saline
> Additive: ionic strength adjusting agent TISAB-11, available from DKK-TOA CORPORATION
> pH: from 5.5 to 6.0
> Temperature during immersion: 37°C
> Time of immersion: 24 hours
> Device for measuring fluorine ion concentration: fluorine ion electrode F-1000, available from DKK-TOA CORPORATION
> Fluorine Concentration

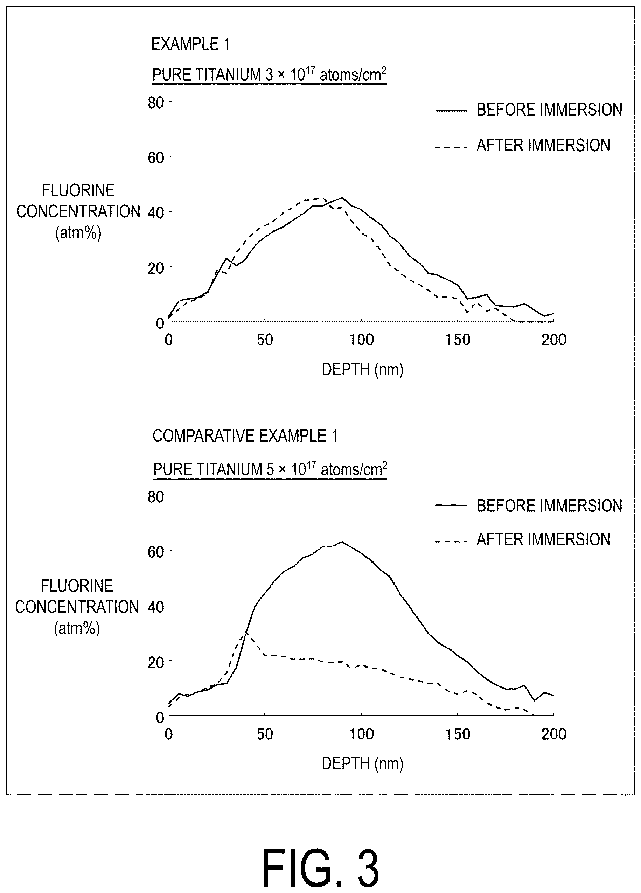

The fluorine concentration in a composite material was measured by SIMS and XPS. Specifically, SIMS measured the fluorine concentration in regions where the fluorine concentration was relatively low and fell below the lower measurement limit of XPS, and XPS measured the fluorine concentration in the other regions. Measurement by XPS was performed at a depth of from 0 to 200 nm. Measurement by SIMS was performed at a depth of from 0 to 1100 nm. Measurement of fluorine concentration was performed on samples before and after immersion in 10 mL of saline at 37°C for 24 hours. FIGs. 3, 8, and 12 only illustrate the measurement results at a depth of from 0 to 200 nm. The depth of 0 nm in FIGs. 3, 8, and 12 represents a surface of a composite material.

Measurement Conditions of XPS

> Analyzer: X-ray photoelectron spectrometer "PHI Quantera II", available from ULVAC-PHI
> X-ray source: monochromatic AlK$\alpha$
> Sputtering ions: Ar$^+$
> Acceleration voltage: 4 kV
> Measurement Conditions of SIMS
> Analyzer: secondary ion mass analyzer "D-SIMS 6650", available from ULVAC-PHI
> Primary ion species: Cs$^+$
> Secondary ion polarity: negative
> Acceleration voltage: 2 kV
> Beam current: 25 nA
> Charge compensation: none
> Raster size: 400 $\mu$m

The thickness T of the surface layer was also measured using SIMS. The surface layer was defined as the region from a surface (depth 0 nm) of the composite material to a depth at which the fluorine concentration was 1 ppm or less.

Fluorine Bonding State

The fluorine bonding state in the composite material was analyzed using XPS narrow scan. The fluorine-bonding state analysis was performed on samples before and after immersion in 10 mL of saline at 37°C for 24 hours.

Antibacterial Property

A film adhesion test using Staphylococcus aureus was performed in accordance with JIS Z 2801. In Example 1 and Comparative Example 1, a film adhesion test was performed using samples before and after immersion in 10 mL of saline at 37°C for 24 hours. In Example 3, a film adhesion test was performed using a sample before immersion in saline. In addition, for the purpose of comparing antibacterial property, a film adhesion test was also performed on a pure titanium sample (untreated titanium) having the same shape but without fluorine ions implanted.

Cytotoxicity

The relative colony formation rate was evaluated by a direct contact method in accordance with ISO 10993-5. The relative colony formation rate refers to a colony formation rate compared to that of a polyethylene sheet as a positive control. In addition, in order to compare cytotoxicity, the relative colony formation rate was also evaluated for a pure titanium sample (untreated titanium) having the same shape but without fluorine ions implanted. The evaluation was performed under the following conditions.

Cells: Chinese Hamster-derived V79 cells
Culture period: 6 days
Counting: After staining the cells, the number of colonies was counted under a stereoscopic microscope.

Comparison between Example 1 and Comparative Example 1

Example 1

Pure titanium (C.P. Grade 2 Titanium) was prepared as a test piece material. The test piece material was formed into a disc-shaped sample having a diameter of 14 mm and a thicknesses of 1 mm. The disk-shaped sample was used as a base material. The base material was subjected to ultrasonic cleaning using ethanol and acetone, and was then vacuum dried in a desiccator. Thereafter, fluorine ions were implanted into one side of the base material (one side of the disc-shaped sample) under the following conditions.

Implantation method: ion beam implantation method
Implantation energy: 40 keV
Implantation dose: $3 \times 10^{17}$ atoms/cm$^2$
This resulted in a composite material of Example 1.

Comparative Example 1

A composite material of Comparative Example 1 was produced in the same manner as in Example 1 except that the implantation dose was changed to $5 \times 10^{17}$ atoms/cm$^2$.

Evaluation Result

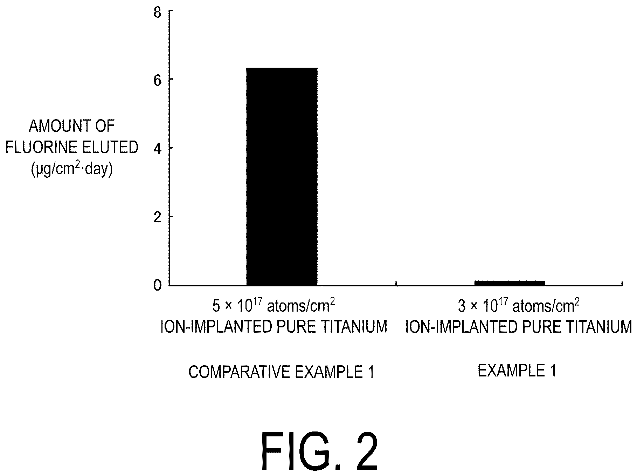

FIG. 2 is a graph illustrating amounts of fluorine eluted in Example 1 and in Comparative Example 1. The amount of fluorine eluted was 6.3 $\mu$g/cm$^2$·day in Comparative Example 1 but 0.1 $\mu$g/cm$^2$ ·day in Example 1. That is, the amount of fluorine eluted was significantly smaller in Example 1 than in Comparative Example 1.

FIG. 3 is a set of graphs illustrating changes in fluorine concentration before and after immersion in saline in Example 1 and in Comparative Example 1. In Comparative Example 1, the peak concentration of fluorine before the immersion was 63.2 atm% while the peak concentration after 24 hours of immersion was reduced to 30.5 atm%. Meanwhile, in Example 1, the peak concentration of fluorine was 45.0 atm% both before and after immersion in saline. That is, it was found that the fluorine concentration in Example 1 was maintained even after 24 hours of immersion in saline. The thicknesses T of the surface layer before the immersion was 740 nm in Comparative Example 1 and was 1070 nm in Example 1 (not illustrated).

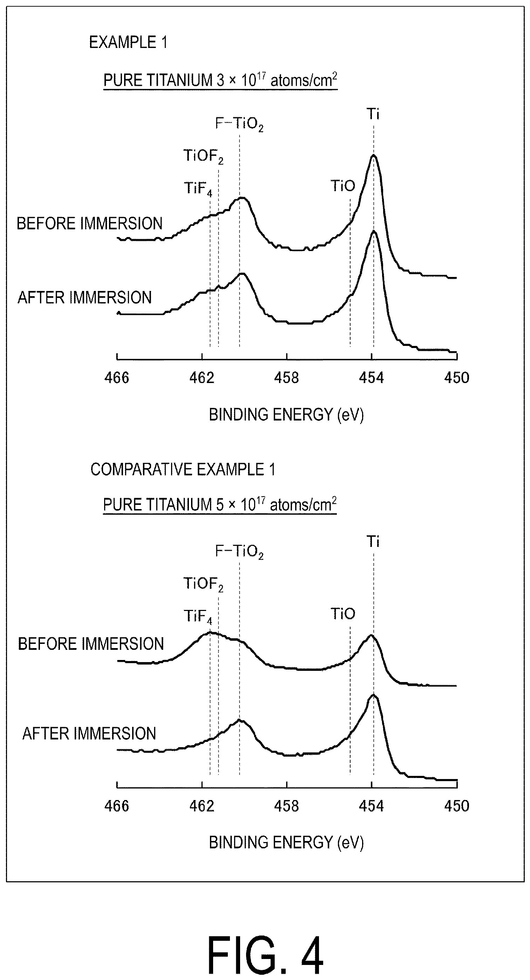

FIG. 4 is a set of graphs illustrating changes in fluorine bonding state of an Ar-sputtered side from a sample surface to a depth of 90 nm before and after immersion in saline in Example 1 and in Comparative Example 1. Because of the fluorine ion implantation, a binding energy attributed to a fluorine compound was recognized in a range of from 460 to 462 eV, in addition to the peak at 453.9 eV attributed to the base material Ti. In Comparative Example 1, before the immersion, the signal intensity at 461.6 eV attributed to TiF$_4$ was the maximum among the fluorine compounds. In the bonding state after the immersion, the peak attributed to TiF$_4$ decreased, and the signal intensity at 460.2 eV attributed to F-TiO$_2$ became the maximum. From this change, it was inferred that TiF$_4$ was in a bonding state in relation to fluorine elution. In Example 1, the peak attributed to F-TiO$_2$ was the largest both before and after the immersion. From the description above, it is clear that the fluorine elution characteristics vary depending on the composition ratio of the fluorine compounds.

Attention was paid to the fluorine compounds detected by the binding energy of Ti2p$_{1/2}$. The composition ratio of these fluorine compounds was inferred from the area ratio of peak separation before the immersion. From the peak areas of F-TiO$_2$ (460.2 eV), TiOF$_2$ (461.2 eV), and TiF$_4$ (461.6 eV), the peak area ratio of TiF$_4$ was calculated in accordance with Equation 1.

Equation 1

$$R_{TiF_4} = A_{TiF_4} \div (A_{TiF_4} + A_{F-TiO_2} + A_{TiOF_2}) \quad \cdots (\text{Equation 1})$$

wherein $R_{TiF_4}$ is a peak area ratio of $TiF_4$, $A_{TiF_4}$ is a peak area of $TiF_4$, $A_{F-TiO_2}$ is a peak area of $F\text{-}TiO_2$, and $A_{TiOF_2}$ is a peak area of $TiOF_2$.

The results are as follows.

## Equation 2

$$\text{Comparative Example 1:} \quad R_{TiF_4} = 0.58$$
$$\text{Example 1:} \quad R_{TiF_4} = 0.34$$

The above proved that rapid elution occurred in Comparative Example 1 in which the peak area ratio of $TiF_4$ measured by XPS was 0.58, and elution was able to be reduced in Example 1 in which the peak area ratio was 0.34. That is, it can be seen that in Example 1, the abundance of the compound of titanium and fluorine did not exceed the abundance of the compound of titanium, fluorine, and oxygen, and thus the elution of fluorine was able to be reduced.

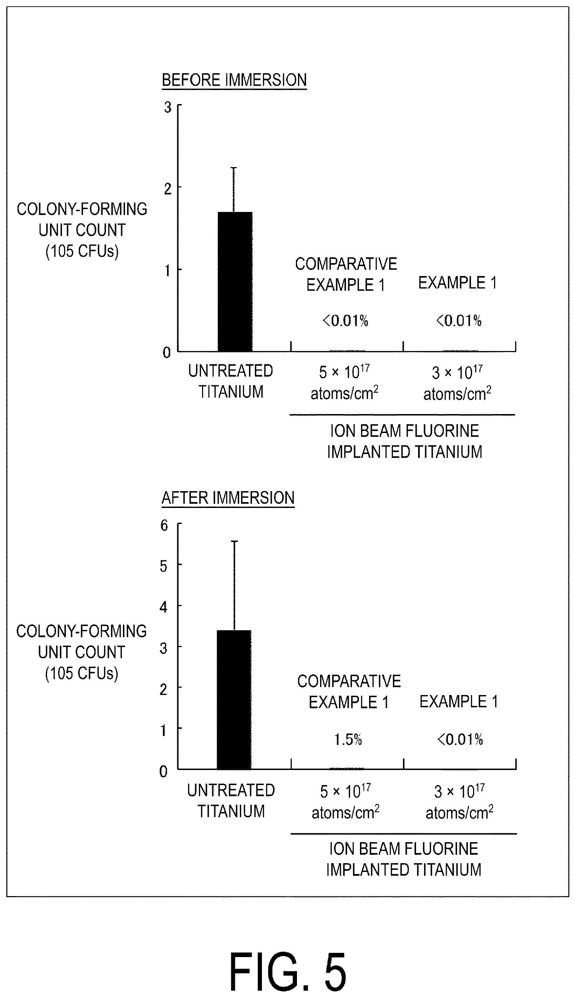

FIG. 5 is a set of graphs illustrating antibacterial properties before and after immersion in saline in untreated titanium as well as in Example 1 and in Comparative Example 1. The measurement results illustrate an average colony-forming unit (CFU) count of three samples. Error bars in the graphs represent standard deviations. The colony-forming unit count before the immersion was 17667 CFUs in untreated titanium, whereas it was less than 10 CFUs (below the detection limit) both in Comparative Example 1 and in Example 1. The colony-forming unit count after the immersion was 340000 CFUs in untreated titanium and 5100 CFUs in Comparative Example 1, whereas it was less than 10 CFUs (below the detection limit) in Example 1.

The results of the measurement revealed that both Comparative Example 1 and Example 1 before the immersion had a colony-forming unit count below the detection limit, exhibiting a high antibacterial effect. When compared to untreated titanium, both Comparative Example 1 and Example 1 statistically significantly reduced the colony-forming unit count to less than 0.01%. In the following description, "statistically significantly" or "statistically significant" means that there is a significant difference at a level of significance of less than 5% as determined by Student's T-test between two groups or by Tukey's multiple comparison test among three or more groups. In Comparative Example 1 after the immersion, a colony-forming unit count of 5100 CFUs was confirmed. Compared to untreated titanium, Comparative Example 1 statistically significantly reduced the colony-forming unit count to 1.5%. However, the antibacterial performance of Comparative Example 1 after the immersion was worse than before the immersion. Meanwhile, in Example 1 after the immersion, the colony-forming unit count was below the detection limit, clearly indicating that a high antibacterial property was maintained.

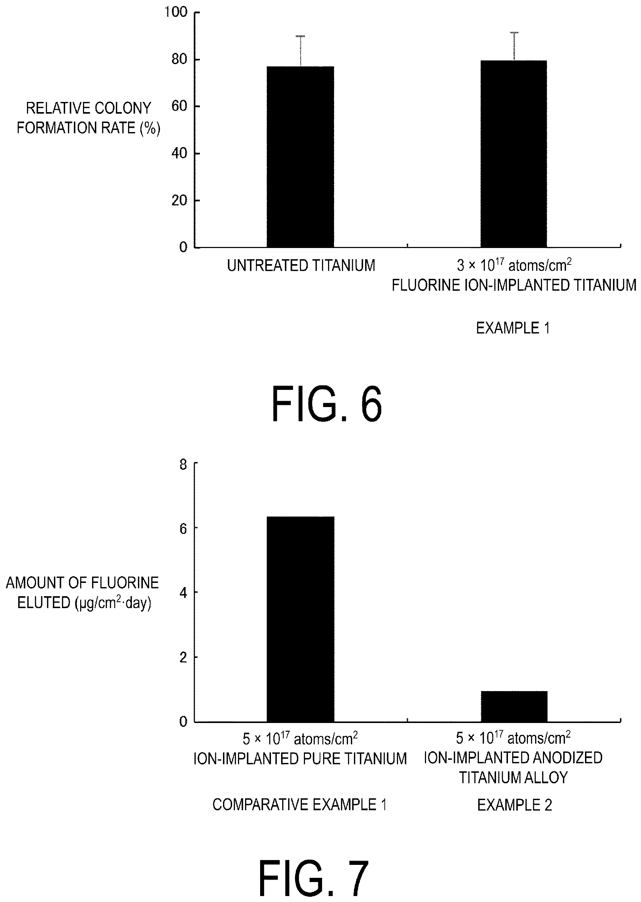

FIG. 6 is a graph illustrating relative colony formation rates in untreated titanium and in Example 1. The measurement results illustrate an average value of the relative colony formation rates of three samples. Error bars in the graphs represent standard deviations. The relative colony formation rate in Example 1 was 80%, and a statistically significant difference compared to that of untreated titanium was not confirmed. Example 1 was found to have no or very little toxicity when tested by the direct contact method.

Comparison between Example 2 and Comparative Example 1

Example 2

A titanium-alloy sheet with a thickness of 1 mm made of Ti-6Al-4V alloy was subjected to anodic oxidation until the titanium-alloy sheet turned blue. The titanium-alloy sheet after anodic oxidation was formed into a disc-shaped sample having a diameter of 14 mm and a thicknesses of 1 mm. The disk-shaped sample was used as a base material. The base material was subjected to ultrasonic cleaning using ethanol and acetone, and was then vacuum dried in a desiccator. Thereafter, fluorine ions were implanted into one side of the base material (one side of the disc-shaped sample) under the following conditions.

Implantation method: ion beam implantation method
Implantation energy: 40 keV
Implantation dose: $5 \times 10^{17}$ atoms/cm$^2$
This resulted in a composite material of Example 2.

Evaluation Result

The results of comparing the composite material of Example 2 with the composite material of Comparative Example 1 described above are presented below. FIG. 7 is a graph illustrating amounts of fluorine eluted in Example 2 and in Comparative Example 1. The amount of fluorine eluted in Example 2 was 0.9 $\mu$g/cm$^2$·day. The amount of fluorine eluted in Example 2 was significantly lower than the 6.3 $\mu$g/cm$^2$·day of fluorine eluted in Comparative Example 1, despite that the fluorine implantation dose in Example 2 was equivalent to that in Comparative Example 1.

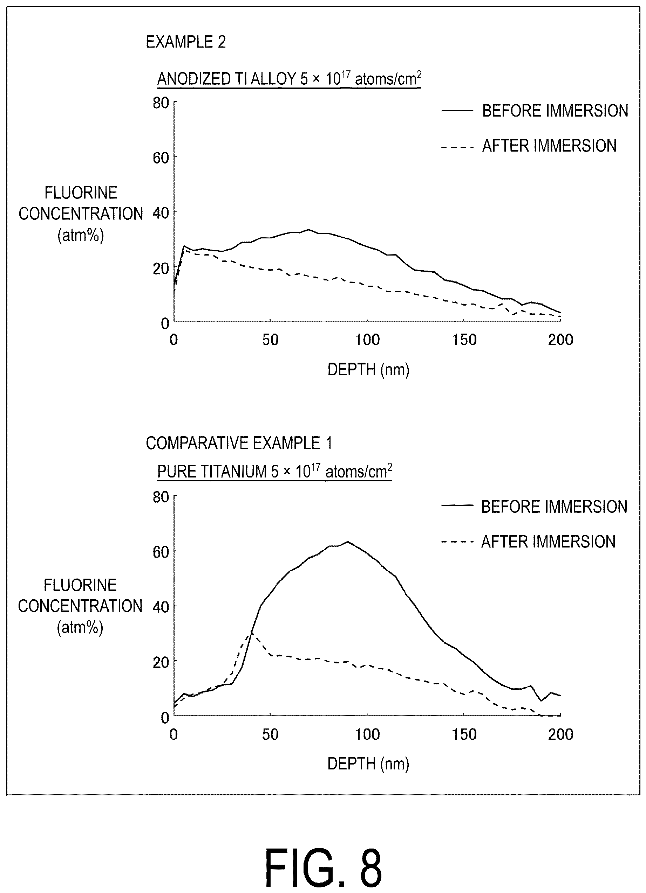

FIG. 8 is a set of graphs illustrating changes in fluorine concentration before and after immersion in saline in Example 2 and in Comparative Example 1. In Example 2, the peak concentration of fluorine before the immersion was 33.4 atm%, and the peak concentration after 24 hours of immersion was 26.5 atm%.

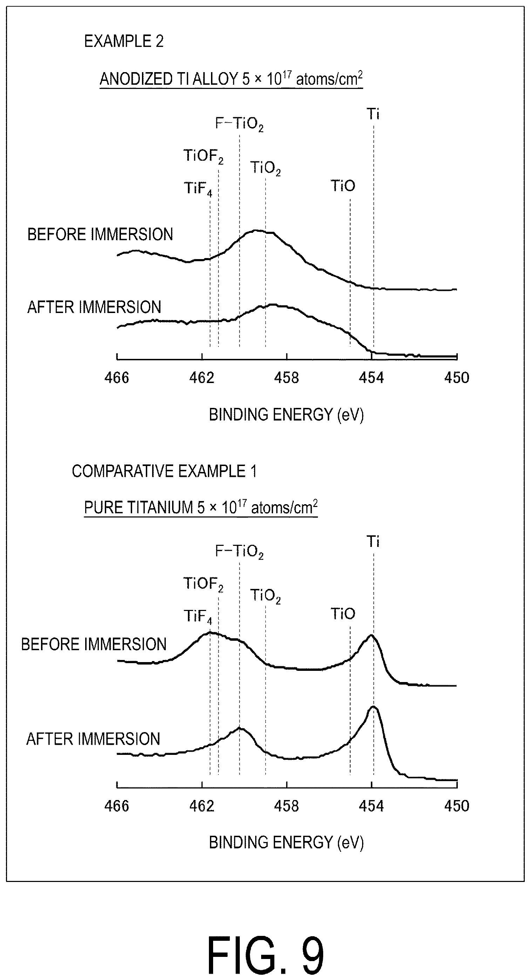

FIG. 9 is a set of graphs illustrating changes in fluorine bonding state before and after immersion in saline in Example 2 and in Comparative Example 1. The changes in Example 2 were regarding an Ar-sputtered side from a sample surface to a depth of 60 nm, and the changes in Comparative Example 1 were regarding an Ar-sputtered side from a sample surface to a depth of 90 nm. In Example 2, the peak at 458.7 eV attributed to $TiO_2$ formed by anodic oxidation and the binding energy attributed to the fluorine compounds in a range of from 460 to 462 eV were confirmed. In Example 2, the intensity at 460.2 eV attributed to $F\text{-}TiO_2$ was the maximum among the fluorine compounds both before and after the immersion. That is, it can be seen that in Example 2, the abundance of the compound of titanium and fluorine does not exceed the abundance of the compound of titanium, fluorine, and oxygen.

Although peak separation was carried out for $F\text{-}TiO_2$ (460.2 eV), $TiOF_2$ (461.2 eV) and $TiF_4$ (461.6 eV), peaks attributable to $TiOF_2$ and $TiF_4$ were not separated. In Example 2, it is conceivable that the fluorine implanted into the oxide film existed mainly as $F\text{-}TiO_2$.

Comparison between Comparative Example 2 and Example 3

Comparative Example 2

Pure titanium (C.P. Grade 2 Titanium) was prepared as a test piece material. The test piece material was formed into a disc-shaped sample having a diameter of 14 mm and a thicknesses of 1 mm. The disk-shaped sample was used as a base material. The base material was subjected to ultrasonic cleaning using ethanol and acetone, and was then vacuum dried in a desiccator. Thereafter, fluorine ions were implanted into the disc-shaped sample by a plasma-based ion implantation method. In accordance with the method described in JP 4568396 B, the conditions for fluorine ion implantation were as follows.

Implantation method: plasma-based ion implantation method
Fluorine source: $C_4F_8$
Implantation energy: 30 keV

This resulted in a composite material of Comparative Example 2.

Example 3

A disc-shaped sample that had been subjected to fluorine ion implantation in the same manner as in Comparative Example 2 was subjected to an oxidation treatment by a heating method under the following conditions.

Atmosphere: air
Rate of temperature rise: 10°C/min
Holding temperature: 300°C
Holding time: 30 mins
Cooling rate: air cooling

This resulted in a composite material of Example 3.

Evaluation Result

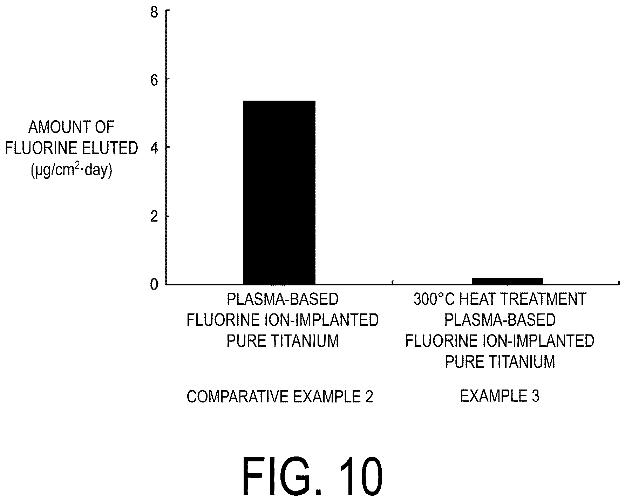

FIG. 10 is a graph illustrating amounts of fluorine eluted in Comparative Example 2 and in Example 3. The amount of fluorine eluted in Comparative Example 2 was 5.3 $\mu$g/cm$^2$·day. The amount of fluorine eluted in Example 3 was 0.2 $\mu$g/cm$^2$·day. That is, Example 3 had a smaller amount of fluorine eluted than that of Comparative Example 2.

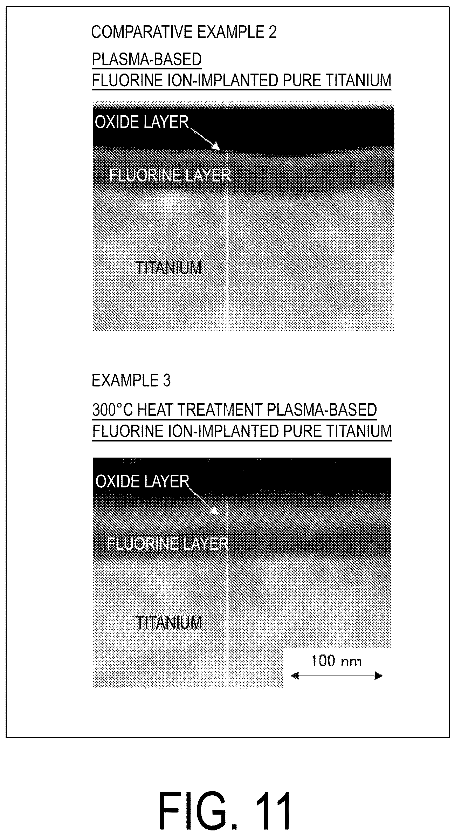

FIG. 11 is a set of cross-sectional views of composite materials in Comparative Example 2 and in Example 3 taken by a transmission electron microscope. An oxide film resulting from natural oxidation was formed on the outermost surface of the composite material in Comparative Example 2, and a fluorine layer containing fluorine located beneath the oxide film was observed. Furthermore, in an observed image, the base material titanium was just beneath the fluorine layer. In Example 3, an oxide layer grown as a result of the oxidation treatment located at the outermost surface was observed. In an observed image, a fluorine layer was beneath the oxide layer, and a titanium was beneath the fluorine layer.

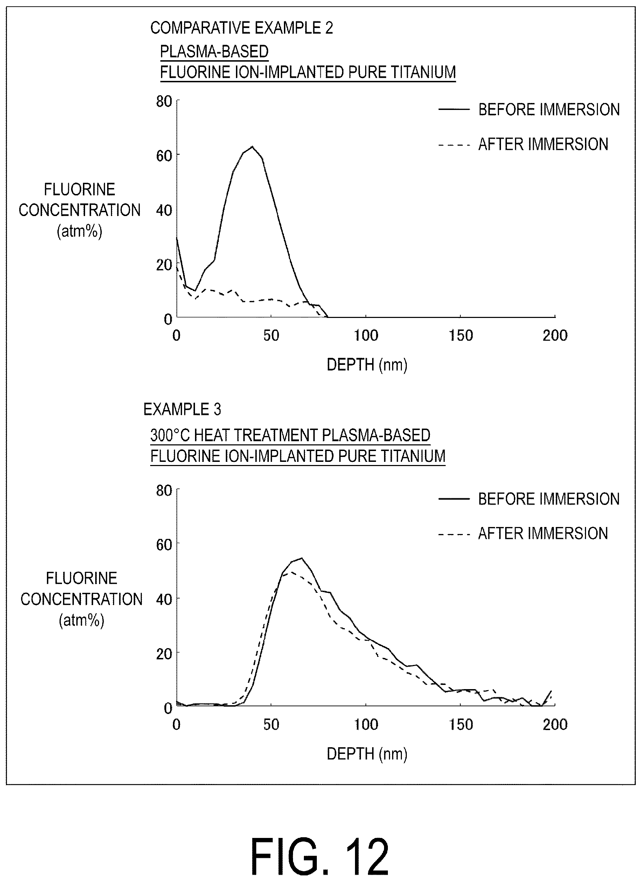

FIG. 12 is a set of graphs illustrating changes in fluorine concentration before and after immersion in saline in Comparative Example 2 and in Example 3. In Comparative Example 2, before the immersion, a peak concentration of fluorine of 63.0 atm% was observed at a position near 40 nm from the surface; however, after 24 hours of immersion, the peak concentration was reduced to 10.1 atm%. In Example 3, the oxide layer formed as a result of the oxidation treatment was observed in a range of from a surface to a depth of 40 nm, and almost no fluorine was detected in this portion. A fluorine distribution having a peak concentration of 54.7 atm% was observed at a position near a depth of 70 nm, which is further below the oxide layer. In Example 3, no significant change was confirmed in the fluorine distribution even after the immersion. That is, it was found that fluorine can be maintained by performing the oxidation treatment.

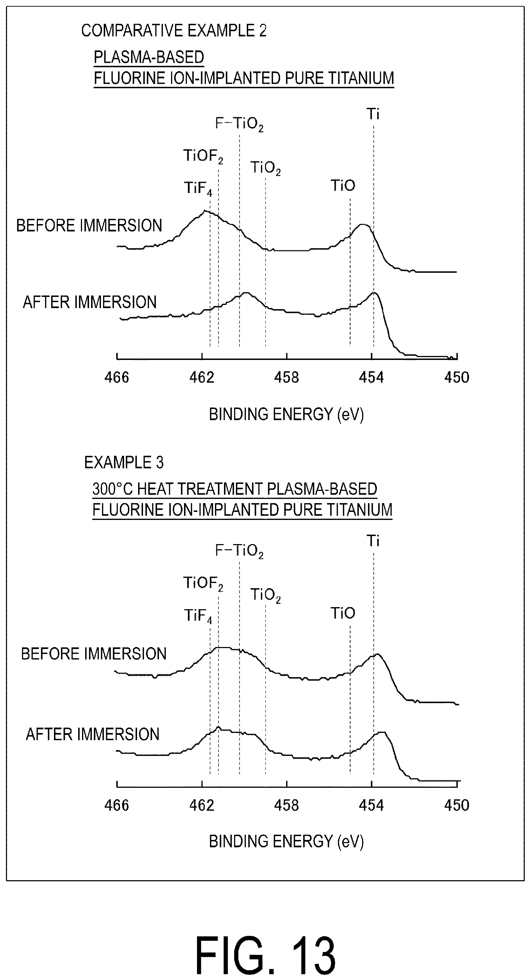

FIG. 13 is a set of graphs illustrating changes in fluorine bonding state before and after immersion in saline in Comparative Example 2 and in Example 3. The changes in Comparative Example 2 were regarding an Ar-sputtered side from a surface to a depth of 50 nm, and the changes in Example 3 were regarding an Ar-sputtered side from a surface to a depth of 60 nm. In Comparative Example 2, the intensity at 461.6 eV attributed to $TiF_4$ was the maximum. In the bonding state after the immersion in Comparative Example 2, the peak attributed to $TiF_4$ decreased, and a peak of 460.2 eV attributed to $F-TiO_2$ was confirmed. The peak attributed to $TiF_4$ decreased in Example 3 compared to that in Comparative Example 2. In Example 3, no significant change was confirmed in the ratio of distribution of the bonding state even after the immersion, and it was conceivable that the composition was able to be maintained.

Attention was paid to the fluorine compounds detected by the binding energy of $Ti2p_{1/2}$. The composition ratio of these fluorine compounds was inferred from the area ratio of peak separation before the immersion. From the peak areas of $F-TiO_2$ (460.2 eV), $TiOF_2$ (461.2 eV), and $TiF_4$ (461.6 eV), the peak area ratio of $TiF_4$ was calculated in accordance with Equation 1 described above. The results are as follows.

$$\text{Equation 3}$$

$$\text{Comparative Example 2:} \quad R_{TiF_4} = 0.56$$
$$\text{Example 3:} \quad R_{TiF_4} = 0.38$$

The above proved that, as in Comparative Example 1, rapid fluorine elution occurred in Comparative Example 2 in which the peak area ratio of $TiF_4$ measured by XPS was 0.56, and that elution was able to be reduced in Example 3 in which the peak area ratio of $TiF_4$ was 0.38. That is, it can be seen that in Example 3, the abundance of the compound of titanium and fluorine did not exceed the abundance of the compound of titanium, fluorine, and oxygen, and thus the elution of fluorine was able to be reduced.

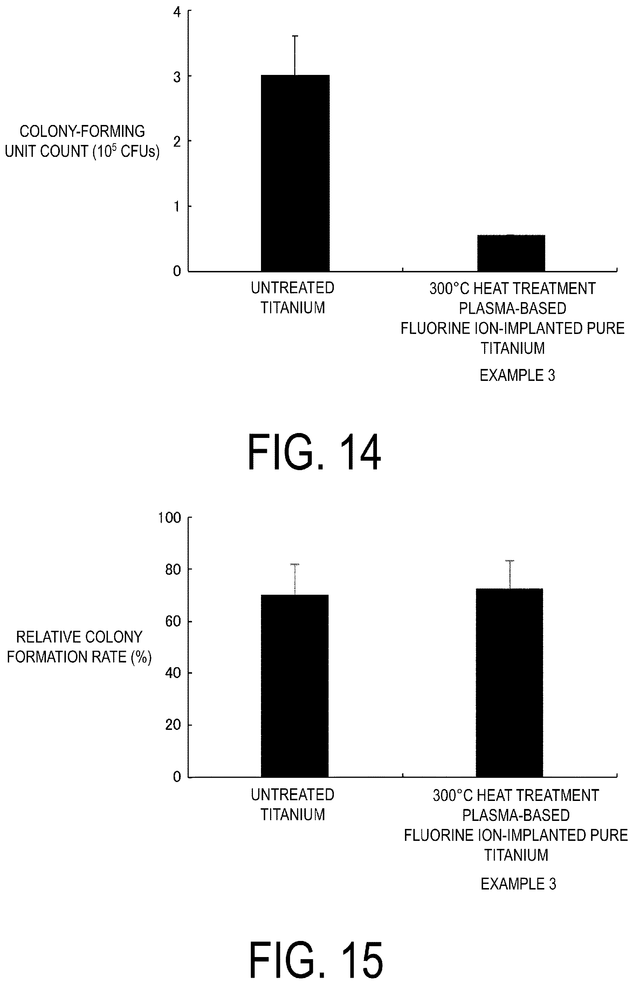

FIG. 14 is a graph illustrating antibacterial properties in untreated titanium and in Example 3. The measurement results illustrate an average colony-forming unit (CFU) count of three samples. Error bars in the graphs represent standard deviations. The colony-forming unit count was 303333 CFUs in untreated titanium, while the colony-forming unit count was 55667 CFUs in Example 3. The measurement results revealed that, compared to untreated titanium, the composite material of Example 3 statistically significantly reduced the colony-forming unit count to 8.5%.

FIG. 15 is a graph illustrating relative colony formation rates in untreated titanium and in Example 3. The measurement results illustrate an average value of the relative colony formation rates of three samples. Error bars in the graphs represent standard deviations. The relative colony formation rate in Example 3 was 78%, and a statistically significant difference compared to that of untreated titanium was not confirmed. Example 3 was found to have no or very little toxicity when tested by the direct contact method.

INDUSTRIAL APPLICABILITY

The present disclosure can be applied to biocompatible implants and fields requiring an antibacterial property.

REFERENCE SIGNS

    100 Dental implant
    101 Fixture
    102 Abutment

103 Artificial tooth

**Claims**

1.  A composite material comprising:

    a base material; and
    a surface layer located on a surface of the base material, the surface layer comprising:

    a compound of titanium and fluorine, and
    a compound of titanium, fluorine, and oxygen,

    wherein an amount of the compound of titanium and fluorine does not exceed an amount of the compound of titanium, fluorine and oxygen,
    wherein a ratio of a peak area of a peak attributed to the compound of titanium and fluorine to a total peak area of the peak attributed to the compound of titanium and fluorine and a peak attributed to the compound of titanium, fluorine and oxygen, calculated from an XPS spectrum resulted from a measurement of the surface layer using X-ray Photoelectron Spectrometry (XPS), is 0.5 or less, and
    an amount of fluorine eluted, as measured by immersion in an acidic solution, is 1 $\mu$g/cm$^2 \cdot$day or less.

2.  The composite material according to claim 1, wherein the compound of titanium, fluorine and oxygen comprises at least one selected from the group consisting of TiOF, $TiO_{2-X}F_{2X}$ (0 < X < 2), fluorine-substituted $F\text{-}TiO_2$, and fluorine-interstitial $F\text{-}TiO_2$.

3.  The composite material according to claim 1 or 2, wherein the compound of titanium and fluorine comprises $TiF_X$ ($1 \leq X \leq 4$).

4.  The composite material according to any one of claims 1 to 3, wherein a maximum value of fluorine concentration exceeds 10 atom%.

5.  The composite material according to any one of claims 1 to 4, wherein a fluorine concentration in the surface layer is 1 ppm or greater.

6.  The composite material according to any one of claims 1 to 5, wherein a thickness of the surface layer is from 20 to 1100 nm.

7.  The composite material according to any one of claims 1 to 6, comprising a compound of titanium and oxygen.

8.  The composite material according to claim 7, wherein the compound of titanium and oxygen comprises $TiO_2$.

9.  The composite material according to any one of claims 1 to 8, wherein the base material comprises pure titanium or a titanium alloy.

10. A biocompatible implant comprising the composite material according to any one of claims 1 to 9.

11. A method for manufacturing a composite material, the method comprising forming the composite material according to claim 1, by one of:

    (a) implanting a fluorine into a base material, an amount of the fluorine being equal or smaller than $3 \times 10^{17}$ atoms/cm$^2$, the base material comprising a metallic titanium;
    (b) implanting a fluorine into a base material, the base material comprising a metallic titanium and comprising a metal oxide film on a surface of the base material;
    (c) reacting a fluorine-implanted base material with oxygen, the base material comprising a metallic titanium; or
    (d) reacting a base material with fluorine and oxygen, the base material comprising a metallic titanium.

12. The method for manufacturing a composite material according to claim 11, wherein, in (a), the amount of the fluorine is larger than $5 \times 10^{16}$ atoms/cm$^2$.

13. The method for manufacturing a composite material according to claim 12, wherein, in (b), the metal oxide film is formed at the surface by a method of atmospheric heat treatment, oxygen plasma treatment, oxygen ion implantation, immersion in an acid solution, or anodic oxidation.

**Patentansprüche**

1. Verbundwerkstoff, umfassend:

   ein Grundmaterial; und
   eine Oberflächenschicht, die sich auf einer Oberfläche des Grundmaterials befindet, wobei die Oberflächenschicht Folgendes umfasst:

   eine Verbindung aus Titan und Fluor und
   eine Verbindung aus Titan, Fluor und Sauerstoff,

   wobei eine Menge der Verbindung aus Titan und Fluor eine Menge der Verbindung aus Titan, Fluor und Sauerstoff nicht übersteigt,
   wobei ein Verhältnis einer Peakfläche eines Peaks, der der Verbindung aus Titan und Fluor zugeordnet ist, zur Gesamtpeakfläche des Peaks, der der Verbindung aus Titan und Fluor zugeordnet ist, und eines Peaks, der der Verbindung aus Titan, Fluor und Sauerstoff zugeordnet ist, berechnet aus einem XPS-Spektrum, das aus einer Messung der Oberflächenschicht unter Verwendung von Röntgen-Photoelektronenspektroskopie (XPS) resultiert, 0,5 oder weniger ist, und
   eine Menge an eluiertem Fluor, die durch Eintauchen in eine saure Lösung gemessen wird, 1 $\mu g/cm^2 \cdot$ Tag oder weniger ist.

2. Verbundwerkstoff gemäß Anspruch 1, wobei die Verbindung aus Titan, Fluor und Sauerstoff mindestens eine umfasst, die aus der Gruppe ausgewählt ist, die aus TiOF, $TiO_{2-X}F_{2X}$ (0 < X < 2), fluorsubstituiertem $F-TiO_2$ und fluorinterstitiellem $F-TiO_2$ besteht.

3. Verbundwerkstoff gemäß Anspruch 1 oder 2, wobei die Verbindung aus Titan und Fluor $TiF_X$ ($1 \leq X \leq 4$) umfasst.

4. Verbundwerkstoff gemäß einem der Ansprüche 1 bis 3, wobei ein Maximalwert der Fluorkonzentration 10 Atom-% überschreitet.

5. Verbundwerkstoff gemäß einem der Ansprüche 1 bis 4, wobei eine Fluorkonzentration in der Oberflächenschicht 1 ppm oder mehr ist.

6. Verbundwerkstoff gemäß einem der Ansprüche 1 bis 5, wobei eine Dicke der Oberflächenschicht von 20 bis 1100 nm ist.

7. Verbundwerkstoff gemäß einem der Ansprüche 1 bis 6, der eine Verbindung aus Titan und Sauerstoff umfasst.

8. Verbundwerkstoff gemäß Anspruch 7, wobei die Verbindung aus Titan und Sauerstoff $TiO_2$ umfasst.

9. Verbundwerkstoff gemäß einem der Ansprüche 1 bis 8, wobei das Grundmaterial reines Titan oder eine Titanlegierung umfasst.

10. Biokompatibles Implantat, das den Verbundwerkstoff gemäß einem der Ansprüche 1 bis 9 umfasst.

11. Verfahren zur Herstellung eines Verbundwerkstoffs, wobei das Verfahren die Bildung des Verbundwerkstoffs gemäß Anspruch 1 umfasst, durch eines aus:

   (a) Implantieren von Fluor in ein Grundmaterial, wobei eine Menge des Fluors gleich oder kleiner als $3 \times 10^{17}$ Atome/cm$^2$ ist, wobei das Grundmaterial metallisches Titan umfasst;
   (b) Implantieren von Fluor in ein Grundmaterial, wobei das Grundmaterial metallisches Titan umfasst und einen Metalloxidfilm auf einer Oberfläche des Grundmaterials umfasst;
   (c) Reagierenlassen eines mit Fluor implantierten Grundmaterials mit Sauerstoff, wobei das Grundmaterial metallisches Titan umfasst; oder

(d) Reagierenlassen eines Grundmaterials mit Fluor und Sauerstoff, wobei das Grundmaterial metallisches Titan umfasst.

12. Verfahren zur Herstellung eines Verbundwerkstoffs gemäß Anspruch 11, wobei in (a) die Menge des Fluors größer als $5 \times 10^{16}$ Atome/cm$^2$ ist.

13. Verfahren zur Herstellung eines Verbundwerkstoffs gemäß Anspruch 12, wobei in (b) der Metalloxidfilm an der Oberfläche durch ein Verfahren der atmosphärischen Wärmebehandlung, der Sauerstoffplasma-Behandlung, der Sauerstoffionenimplantation, des Eintauchens in eine saure Lösung oder der anodischen Oxidation gebildet wird.

**Revendications**

1. Matériau composite comprenant :

un matériau de base ; et
une couche superficielle située sur une surface du matériau de base, la couche superficielle comprenant :

un composé de titane et de fluor, et
un composé de titane, de fluor et d'oxygène,
dans lequel une quantité du composé de titane et de fluor ne dépasse pas une quantité du composé de titane, de fluor et d'oxygène,
dans lequel un rapport entre une surface d'un pic attribué au composé de titane et de fluor et une surface totale du pic attribué au composé de titane et de fluor et d'un pic attribué au composé de titane, fluor et oxygène, calculé à partir d'un spectre XPS résultant d'une mesure de la couche superficielle en utilisant une spectrométrie photoélectronique à rayons X (XPS), est égal ou inférieur à 0,5, et
une quantité de fluor éluée, telle que mesurée par immersion dans une solution acide, est égale ou inférieure à 1 $\mu$g/cm$^2$·jour.

2. Matériau composite selon la revendication 1, dans lequel le composé de titane, de fluor et d'oxygène comprend au moins un élément choisi dans le groupe constitué par TiOF, $TiO_{2-X}F_{2X}$ (0 < X < 2), F-$TiO_2$ substitué par du fluor et F-$TiO_2$ interstitiel fluoré.

3. Matériau composite selon la revendication 1 ou 2, dans lequel le composé de titane et de fluor comprend du $TiF_X$ ($1 \leq X \leq 4$).

4. Matériau composite selon l'une quelconque des revendications 1 à 3, dans lequel une valeur maximale de la concentration en fluor dépasse 10 % en atomes.

5. Matériau composite selon l'une quelconque des revendications 1 à 4, dans lequel une concentration en fluor dans la couche superficielle est égale ou supérieure à 1 ppm.

6. Matériau composite selon l'une quelconque des revendications 1 à 5, dans lequel l'épaisseur de la couche superficielle est de 20 à 1100 nm.

7. Matériau composite selon l'une quelconque des revendications 1 à 6, comprenant un composé de titane et d'oxygène.

8. Matériau composite selon la revendication 7, dans lequel le composé de titane et d'oxygène comprend du $TiO_2$.

9. Matériau composite selon l'une quelconque des revendications 1 à 8, dans lequel le matériau de base comprend du titane pur ou un alliage de titane.

10. Implant biocompatible comprenant le matériau composite selon l'une quelconque des revendications 1 à 9.

11. Procédé de fabrication d'un matériau composite, le procédé comprenant la formation du matériau composite selon la revendication 1, par l'une des :

(a) l'implantation de fluor dans un matériau de base, une quantité de fluor étant égale ou inférieure à $3 \times 10^{17}$ atomes/cm$^2$, le matériau de base comprenant du titane métallique ;

(b) l'implantation d'un fluor dans un matériau de base, le matériau de base comprenant un titane métallique et comprenant un film d'oxyde métallique sur une surface du matériau de base ;

(c) la réaction d'un matériau de base implanté au fluor avec de l'oxygène, le matériau de base comprenant un titane métallique ; ou

(d) la réaction d'un matériau de base avec du fluor et de l'oxygène, le matériau de base comprenant un titane métallique.

12. Procédé de fabrication d'un matériau composite selon la revendication 11, dans lequel, dans (a), la quantité de fluor est supérieure à $5 \times 10^{16}$ atomes/cm$^2$.

13. Procédé de fabrication d'un matériau composite selon la revendication 12, dans lequel, dans (b), le film d'oxyde métallique est formé à la surface par un procédé de traitement thermique atmosphérique, de traitement au plasma d'oxygène, d'implantation d'ions oxygène, d'immersion dans une solution acide ou d'oxydation anodique.

# FIG. 1

FIG. 2

EXAMPLE 1

PURE TITANIUM 3 × 10$^{17}$ atoms/cm$^2$

COMPARATIVE EXAMPLE 1

PURE TITANIUM 5 × 10$^{17}$ atoms/cm$^2$

# FIG. 3

EXAMPLE 1

PURE TITANIUM $3 \times 10^{17}$ atoms/cm$^2$

COMPARATIVE EXAMPLE 1

PURE TITANIUM $5 \times 10^{17}$ atoms/cm$^2$

# FIG. 4

BEFORE IMMERSION

AFTER IMMERSION

# FIG. 5

RELATIVE COLONY FORMATION RATE (%)

UNTREATED TITANIUM

$3 \times 10^{17}$ atoms/cm$^2$
FLUORINE ION-IMPLANTED TITANIUM

EXAMPLE 1

# FIG. 6

AMOUNT OF FLUORINE ELUTED ($\mu$g/cm$^2$·day)

$5 \times 10^{17}$ atoms/cm$^2$
ION-IMPLANTED PURE TITANIUM

$5 \times 10^{17}$ atoms/cm$^2$
ION-IMPLANTED ANODIZED TITANIUM ALLOY

COMPARATIVE EXAMPLE 1

EXAMPLE 2

# FIG. 7

FIG. 8

EXAMPLE 2

ANODIZED TI ALLOY 5 × $10^{17}$ atoms/cm$^2$

BINDING ENERGY (eV)

COMPARATIVE EXAMPLE 1

PURE TITANIUM 5 × $10^{17}$ atoms/cm$^2$

BINDING ENERGY (eV)

FIG. 9

FIG. 10

COMPARATIVE EXAMPLE 2

PLASMA-BASED
FLUORINE ION-IMPLANTED PURE TITANIUM

EXAMPLE 3

300°C HEAT TREATMENT PLASMA-BASED
FLUORINE ION-IMPLANTED PURE TITANIUM

# FIG. 11

FIG. 12

FIG. 13

**FIG. 14**

**FIG. 15**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 4568396 B


**Non-patent literature cited in the description**

- **LIU H Y et al.** Effect of fluoride-ion implantation on the biocompatibility of titanium for dental applications. *APPLIED SURFACE SCIENCE*, 15 August 2008, vol. 254 (20), 6305-6312